(19) Europäisches Patentamt / European Patent Office / Office européen des brevets

(11) **EP 3 490 527 B1**

(12) **EUROPEAN PATENT SPECIFICATION**

(45) Date of publication and mention
of the grant of the patent:
**07.06.2023  Bulletin 2023/23**

(21) Application number: **17742446.2**

(22) Date of filing: **26.07.2017**

(51) International Patent Classification (IPC):
*A61K 9/00* $^{(2006.01)}$   *A61K 31/00* $^{(2006.01)}$
*A61K 47/38* $^{(2006.01)}$   *A61K 47/36* $^{(2006.01)}$
*A61K 47/10* $^{(2017.01)}$   *A61P 17/02* $^{(2006.01)}$
*A61P 35/00* $^{(2006.01)}$   *C07K 16/24* $^{(2006.01)}$
*A61K 31/728* $^{(2006.01)}$   *A61P 29/00* $^{(2006.01)}$
*A61K 39/395* $^{(2006.01)}$   *A61K 31/4745* $^{(2006.01)}$
*A61K 39/00* $^{(2006.01)}$

(52) Cooperative Patent Classification (CPC):
(C-Sets available)
**A61K 9/0031; A61K 31/4745; A61K 31/728;**
**A61K 39/39591; A61K 47/10; A61K 47/36;**
**A61K 47/38; A61P 17/02; A61P 29/00;**
**A61P 35/00; C07K 16/241;** A61K 2039/505  (Cont.)

(86) International application number:
**PCT/EP2017/068876**

(87) International publication number:
**WO 2018/019881 (01.02.2018 Gazette 2018/05)**

(54) **BIOADHESIVE PLATFORM TO PERFORM BIOACTIVE TREATMENT**

BIOADHÄSIVE PLATTFORM ZUR DURCHFÜHRUNG EINER BIOAKTIVEN BEHANDLUNG

PLATE-FORME BIOADHÉSIVE POUR EFFECTUER UN TRAITEMENT BIOACTIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **27.07.2016  EP 16382365**

(43) Date of publication of application:
**05.06.2019  Bulletin 2019/23**

(73) Proprietors:
• **Fundació Institut d'Investigació en Ciències de la
Salut Germans Trias i Pujol
08916 Badalona (ES)**
• **Consorcio Centro de Investigación Biomédica en
Red
M.P.
28029 Madrid (ES)**

(72) Inventors:
• **LORENZO-ZÚÑIGA GARCÍA, Vicente María
08016 Barcelona (ES)**
• **BARTOLÍ SOLÉ, Ramon
08001 Barcelona (ES)**
• **BOIX VALVERDE, Jaume
08025 Barcelona (ES)**

(74) Representative: **ZBM Patents - Zea, Barlocci &
Markvardsen
Rambla Catalunya, 123
08008 Barcelona (ES)**

(56) References cited:
**WO-A1-00/19981       WO-A1-2016/135219
US-A1- 2006 280 797**

- **WANG LU-LU ET AL: "Development of rectal delivered thermo-reversible gelling film encapsulating a 5-fluorouracil hydroxypropyl-[beta]-cyclodextrin complex", CARBOHYDRATE POLYMERS, APPLIED SCIENCE PUBLISHERS, LTD. BARKING, GB, vol. 137, 19 October 2015 (2015-10-19), pages 9-18, XP029344662, ISSN: 0144-8617, DOI: 10.1016/J.CARBPOL.2015.10.042**
- **KIBBE A H ED - KIBBE A H (ED): "Handbook of pharmaceutical excipients, Carbomer, Glycerin, Isopropyl Myristate, Isopropyl Palmitate, Methylcellulose, Propylene Glycol", 1 January 2000 (2000-01-01), HANDBOOK OF PHARMACEUTICAL EXCIPIENTS, AMERICAN PHARMACEUTICAL ASSOC. [U.A.], WASHINGTON, DC; US, PAGE(S) 79 - 82,220, XP002511422, ISBN: 978-0-85369-381-9 Methylcellulose - monography**

(52) Cooperative Patent Classification (CPC): (Cont.)

C-Sets
A61K 31/4745, A61K 2300/00;
A61K 31/728, A61K 2300/00

## Description

This application claims the benefit of European Patent Application EP16382365.1 filed on 27.07.2016.

The present invention relates to compositions comprising hyaluronic acid, and two thermoreversible adhesive agents, being one of them a poloxamer, that may be used as a drug delivery system for the local delivery of active agents to the gastrointestinal tract. It further relates to its uses in medicine, especially as endoscopic shield to treat gastrointestinal cancers and inflammatory diseases. It also relates to injection devices comprising the said compositions, and to kits comprising the injection devices and delivery devices suitable to be coupled to the injection devices.

## Background Art

Endoscopy is a minimally invasive procedure that allows diagnosing conditions inside the gastrointestinal, respiratory or urinary tract, by means of an endoscope, which is inserted through a body passageway. Advances in endoscopic medicine have led to the development of therapeutic endoscopy that enables physicians to treat numerous conditions using endoscopic techniques such as the removal of polyps and early tumors.

The expansion of the indications of advanced therapeutic endoscopic techniques, such as endoscopic mucosal resection (EMR) and endoscopic submucosal dissection (ESD), to include early gastrointestinal cancers, has become routine and has enable extensive resection. This has reduced the need for surgical intervention. However, the appearance of recurrence after the resection is often difficult to manage, requiring risky endoscopic techniques. Otherwise, the presence of isolated symptomatic mucosal lesions, despite medical therapy, is common in inflammatory diseases.

CA2703807 relates to a composite aqueous hydrogel comprising hyaluronic acid, methylcellulose and dispersed polymeric hydrophobic micro and/or nanoparticles. The composite may be injectable, and in the absence of a therapeutic agent it may be used as a bulking agent for reconstructive and cosmetic surgery. The polymeric micro and/or nanoparticles may encapsulate at least one therapeutic agent e.g. for the treatment of spinal cord injury, in which case each therapeutic agent exhibits a linear sustained release rate that can be tuned or altered by selecting the appropriate polymer formulation of the micro and/or nanoparticles. According to this document, the stability of the hydrogel with these polymeric micro and/or nanoparticles is enhanced when compared to the stability of the hydrogel alone described in US20060280797. However, the process for preparing the composite disclosed in CA2703807 requires the preparation of polymeric micro and/or nanoparticles by single or double emulsion methods followed by solvent removal techniques such as extraction, evaporation or spray drying. Further, the particle size of the polymeric micro and/or nanoparticles needs to be tightly controlled. This makes this process expensive and time consuming.

Wang et al (Carbohydrate Polymers 2015, vol. 137, 9-18) discloses a 5-Fluorouracil (5FU) formulation for rectal application in colorectal cancer. It describes the effects of an inclusion complex of 5FU with Hydroxypropyl-$\beta$-Cyclodextrin (HP-$\beta$-CD) encapsulated with thermo-reversible gelling films formed from a matrix of Poloxamer407/Poloxamer188 and a bio-adhesive polymer (Carbopol, polycarbophil, HPMC, sodium carboxymethyl starch, HA/SH (hyaluronate), or sodium alginate).

Therefore, taking into account the large number of therapeutic endoscopy procedures carried out today, and the increasing incidence of inflammatory diseases and cancers such as inflammatory bowel disease, inflammatory colitis or colorectal cancer, it is imperative to assess a novel scenario for endoscopic therapy. In this sense, there is a need to develop of a bioadhesive and bioactive platform to deliver local treatments, which reduces or avoid post-resection recurrence and solves refractory mucosal lesions by inducing mucosal healing.

## Summary of Invention

The inventors have developed new safe and stable pharmaceutical or veterinary compositions that comprise hyaluronic acid or a salt thereof as therapeutically active agent, and two thermoreversible adhesive agents, being one of them a poloxamer, wherein the poloxamer and the hyaluronic acid or salt are present in a specific ratio. Thanks to the properties of the compositions of the invention they can be used to perform long term bioactive treatment.

In the absence of any further active agent, the compositions of the invention (also referred herein to base compositions) are suitable for the topical treatment of mucosal lesions and/or for the prevention of complications derived from mucosal lesions by providing good healing properties.

The base composition of the invention may also be used as a platform for the local and sustained delivery of active agents, for example monoclonal and polyclonal antibodies, anti-angyogenic or cytostatic agents, and anti-inflammatory medicines, to the gastrointestinal tract, for example to prevent tumor recurrence (e.g. colorectal cancer) after resection, or reducing inflammation in patients with inflammatory bowel disease or inflammatory colitis.

Unlike in the prior art delivery system described in CA2703807, in order to achieve the desired stability in the composition of the invention having a specific ratio poloxamer:hyaluronic acid or salt, there is no need that the poloxamer is in the form of micro and/or nanoparticles that encapsulate the active agent to be delivered when it is present in the composition. This greatly facilitates the production process of the compositions of the invention and makes them more versatile: the preparation process is simpler and contains fewer steps.

Furthermore, this allows that any person (not only the producer of the platform composition), for example the medical staff who is administering the composition to a patient, is able to add the drug needed for the treatment of the pathology of interest to a pre-prepared base composition comprising the hyaluronic acid or salt, the adhesive agent, and the poloxamer before its administration.

Additionally, the composition of the invention has further advantages: it is biodegradable and bioactive (even when it has no additional active agents). Its thermoreversible properties make it easy to apply through the endoscope without requiring any special or complex devices. Moreover, due to its viscosity and adhesion properties at body temperature, it has the ability to remain adhered to the affected area for a long period of time, thus facilitating the pharmacologic activity of the administered active agent.

Moreover, as illustrated in the examples, the stability and integrity of the compositions of the invention is also improved, in particular on the gastrointestinal mucosa, which is colonized by microbiota, with respect to other compositions not containing two thermoreversible adhesive agents as defined herein. Thus, the compositions of the invention show an extended half-life against the effect of enzymes present in the gastrointestinal tract.

Therefore, a first aspect of the present invention relates to a base pharmaceutical or veterinary composition suitable for the delivery of active agents comprising:

a) from 0.25 to 1.5 wt% a hyaluronic acid or a pharmaceutically or veterinary acceptable salt thereof as active agent, and
b) from 10 to 25 wt% of two thermoreversible adhesive agents,

wherein one of the adhesive agents is a poloxamer and the other is methyl cellulose,
wherein thermoreversible means that the adhesive agent is capable to form a composition that is liquid at room temperature and a gel at body temperature,
in the absence of any further active agent,
wherein the weight ratio between the poloxamer and the hyaluronic acid or its salt is from 60:1 to 10:1; and
wherein all percentages are expressed with respect to the total weight of the composition, provided that the sum of the amounts of the components is equal to or less than 100%.

As mentioned above, the base composition as defined above is useful fin the treatment of topical mucosal lesions, in particular of the gastrointestinal mucosa, and/or for preventing complications derived from such lesions.

Therefore, another aspect of the invention relates to a composition suitable for the delivery of active agents as defined above for use as a medicament.

Another aspect of the invention relates to a composition suitable for the delivery of active agents as defined above for use in the topical treatment of mucosal lesions and/or for the prevention of complications derived from mucosal lesions. This aspect relates to the use of hyaluronic acid or a pharmaceutically or veterinary acceptable salt thereof for the manufacture of a composition suitable for the delivery of active agents as defined above for the topical treatment of mucosal lesions and/or for the prevention of complications derived from mucosal lesions.

As mentioned above, the composition suitable for the delivery of active agents (also referred herein to as base composition) may also be used as a bioadhesive platform for the local delivery of active agents.

Thus, another aspect of the invention relates to a pharmaceutical or veterinary composition comprising the base composition as previously defined and a therapeutically effective amount of one or more further active agents; with the condition that the further active agent is other than a non-absorbable antibiotic, wherein non-absorbable means that the antibiotic is capable of providing activity only locally in the gut. Furthermore, the non-absorbable antibiotic has a negligible systemic absorption.

The compositions of the invention, whether they contain one or more further therapeutically active agents or not, may be administered through and endoscope by an appropriate delivery device.

Thus, another aspect of the invention relates to an injection device comprising the base composition or the composition further comprising one or more further active agents as defined above.

Another aspect of the invention relates to a kit comprising the injection device as defined above, and a delivery device suitable to be coupled to the injection device.

**Brief Description of Drawings**

FIG. 1 shows the evolution of the viscosity of the hydrogel of Example 1 of the invention according to the speed of shear.

FIG. 2 shows the evolution of the viscosity of the hydrogel of Example 2 of the invention according to the speed of shear.

FIG. 3 shows the evolution of the viscosity of the hydrogel of Example 3 of the invention according to the speed of shear.

FIG. 4 shows the release of indigo carmine in PBS medium over time (hours) from the composition of example 1 (diamonds), compared to control PBS (crosses), and two negative controls: the composition of example 1 without indigo carmine (squares) and PBS without indigo carmine (circles).

FIG. 5 shows the pressure needed to flush the composition of example 1 (C), compared to saline (A), comparative composition 1 (B), and comparative composition 2 (D).

FIG. 6 shows the half-life of the composition of example 1 (A, plate 1), comparative composition 3 (without Pluronic F127) (B, plate 2), and comparative composition 4 (without methylcellulose) (C, plate 3) in a degradation test using in plates seeded with colonic lavage.

**Detailed description of the invention**

Unless otherwise stated, all percentages mentioned herein regarding the components of the composition are expressed in weight with respect to the total weight of the composition, provided that the sum of the amounts of the components is equal to 100%.

The present invention discloses pharmaceutical or veterinary compositions comprising hyaluronic acid or a salt thereof as active agent, and two thermoreversible adhesive agents as carriers, being one of them a poloxamer, and the other is methyl cellulose, wherein the weight ratio between the poloxamer and the hyaluronic acid or its salt is from 60:1 to 10:1.

In one particular embodiment, optionally in combination with one or more features of the various embodiments described above or below, the weight ratio between the poloxamer and the hyaluronic acid or its salt is from 60:1 to 20:1, more particularly from 50:1 to 30:1, more particularly is from 45:1 to 35:1, and even more particularly about 40.

For the purposes of the present invention, the term "base composition" is used to define a composition suitable for the delivery of active agents which, except hyaluronic acid or its salt, it does not contain any further active agent. By contrast, the expression "composition further comprising one or more further active agents" refers to a composition comprising one or more further therapeutically active agents in addition to a hyaluronic acid or its salt.

As mentioned above, the compositions of the invention show suitable viscosity and adhesion properties. In particular, when the composition contacts a mucosa, a tissue or an organ at body temperature, it has the consistency of a gel, and has the ability to remain adhered to the affected area for a long period of time.

As used herein, "viscosity" refers to a measure of the resistance of a fluid to deform under shear stress and describe the fluid's internal resistance to flow and can be measured as a function of the shear rate by using a rheometer. For example, the rheological test may be carried out in a Haake device RheoStress equipped with a C60 / 1°Ti sensor and a "gap set" 0.053 mm, a rotation ramp from 0 to 300 s-1 for 30 seconds. For each trial the evolution of viscosity ($\eta$) of the sample according to the speed shear ($\gamma$) to 20 and 40 can be measured.

The term "adhesion" as used herein refers to the ability of the compositions of the invention to bind to the site of topical application or administration, e.g. mucoses, upon contact, by both chemical and physical means, whereby when they are brought into contact work must be done in order to separate them. The adhesion can be measured by a texture analyser TA.XT Plus. For example, a 40-mm (diameter) disk can be compressed into the gel and redrawn. The method settings, including speed rate at 1 mm/s and distance (depth of the insertion) of 9-mm can be assessed at the desired temperature, e.g. at 22 °C or at 37 °C. The adhesion is measured in mN/s units. The more negative the value in mN/s, the more adhesive the composition will be. Thus, for example a composition showing a measurement value of -100 mN/s is more adhesive than a composition showing a lower measurement value of e.g., -50 mN/s.

In one particular embodiment, optionally in combination with one or more features of the various embodiments described above or below, the adhesion of the composition at body temperature is equal to or lower than -20 mN/s, more particularly from -50 to -4000 mN/s, more particularly from -100 to -4000 mN/s, more particularly from -1000 to -4000 mN/s (as measured by the method described above). When the composition shows the above adhesion values at body temperature, it has the advantage that it remains adhered to mucosa for a longer period of time.

In another embodiment, optionally in combination with one or more features of the various embodiments described above or below, the viscosity of the composition at body temperature is from 0.5 to 7000 Pa·s, more particularly from 1 to 6500 Pa·s (as measured by the method described above). When the composition shows the above viscosity values, it forms a particularly thick film, more particularly a film with a thickness from 0.5 to 5 mm, as opposed to a thin film, e.g. when applied to the mucosa. This has the advantage that it further improves the physiological healing process of the mucosal lesion.

In another embodiment, optionally in combination with one or more features of the various embodiments described above or below, the adhesion of the composition at body temperature is equal to or lower than -20 mN/s, more particularly from -50 to -4000 mN/s, more particularly from -100 to -4000 mN/s, more particularly from -1000 to -4000 mN/s; and the viscosity of the composition at body temperature is from 0.5 to 7000 Pa·s, more particularly from 1 to 6500 Pa·s, more particularly from 1000 to 6500 Pa s.

The compositions of the invention are thermoreversible. For the purpose of the present invention, the term "thermoreversible" or equivalent expressions thereof such as "thermally reversible" applied to the composition means that it exhibits reverse thermogellation, i.e., it undergoes a change in viscosity when the temperature varies. Thus, the composition is liquid at room temperature and forms a gel at body temperature. The liquid state at room temperature facilitates the administration of the composition when it is to be administered e.g. to the gastrointestinal mucosa, by using an appropriate injection device, such as for example a syringe or a jet injector, coupled to a delivery device or system, such as a catheter, which can be introduced via an endoscope. When the composition comes into contact with the mucosa at body temperature, its viscosity increases to a higher viscosity state, hence acquiring the consistency of a gel. This has the advantage that the composition remains on the surface of the affected area.

Thus, in one particular embodiment, in combination with one or more features of the various embodiments described above or below, the viscosity of the composition at body temperature is higher than at room temperature, more particularly the viscosity of the composition at body temperature is from 0.5 to 7000 Pa s, more particularly from 1 to 6500 Pa s, more particularly from 1000 to 6500 Pa s, higher than the viscosity of the composition at room temperature.

In another embodiment, optionally in combination with one or more features of the various embodiments described above or below, the adhesion of the composition at body temperature is higher than at room temperature. This means that, in this embodiment, the adhesion value in mN/s of the composition at body temperature is more negative than the adhesion value in mN/s of the composition at room temperature. More particularly, the adhesion of the composition at body temperature is from -20 to -4000 mN/s (in absolute value), more particularly from -50 to -4000 mN/s (in absolute value), more particularly from -100 to -4000 mN/s (in absolute value), more particularly from -1000 to -4000 (in absolute value), higher than the adhesion of the composition at room temperature.

For the purposes of the invention, room temperature refers to a temperature in the range from 20 to 25 °C, and body temperature refers to a temperature in the range from 35 to 42 °C.

As mentioned above, the compositions of the invention comprise a hyaluronic acid or a pharmaceutically or veterinary acceptable salt thereof. Hyaluronic acid (HA) is a naturally occurring anionic non-sulfated glycosaminoglycan distributed widely throughout connective, epithelial, and neural tissues and part of the extracellular matrix. It consists of multiple repeating disaccharide units of N-acetyl-D-glucosamine and D-glucuronic acid. HA plays an important role in tissue repair by its proliferative and immunomodulatory effect inducing tissue repair promoting healing re-epitelisation instead of scaring.

There is no limitation on the type of the hyaluronic acid salt that can be used, provided that they are pharmaceutically or veterinary acceptable when used for therapeutic purposes. The term "pharmaceutically or veterinary acceptable salt", embraces salts commonly used such as e.g. alkali metal salts. The preparation of hyaluronic acid pharmaceutically acceptable salts can be carried out by methods known in the art. Hyaluronic acid and its salts may differ in some physical properties but they are equivalent for the purposes of the present invention.

Non-limiting examples of pharmaceutically or veterinary acceptable salts include inorganic salts such as the sodium, magnesium, potassium, zinc, cobalt salts, and the like, as well as organic salts such as the tetrabutylammonium salt, and the like. In one particular embodiment, optionally in combination with one or more features of the various embodiments described above or below, the composition comprises hyaluronic acid or hyaluronic acid sodium salt, more particularly hyaluronic acid sodium salt.

In one particular embodiment, optionally in combination with one or more features of the various embodiments described above or below, the hyaluronic acid or its pharmaceutically or veterinary acceptable salt is present in an amount from 0.3 to 0.8%, more particularly is about 0.4% by weight (wt%) with respect to the total weight of the composition.

In another particular embodiment, optionally in combination with one or more features of the various embodiments described above or below, the hyaluronic acid or a pharmaceutically acceptable salt thereof has a weight average molecular weight (Mw) from $1.5 \times 10^6$ to $4 \times 10^6$ Daltons, more particularly from $1.7 \times 10^6$ to $2 \times 10^6$ Daltons.

The compositions of the invention also comprise two thermoreversible adhesive agents, being one of them a poloxamer and the other is methyl cellulose. The adhesive agents act as carriers in the pharmaceutical and veterinary compositions as defined herein.

Poloxamers, also known as pluronic compounds, are nonionic triblock copolymers composed of a central hydrophobic chain of polyoxypropylene (polypropylene oxide)) (PPO) flanked by two hydrophilic chains of polyoxyethylene (poly(ethylene oxide)) (PEO). In one embodiment of the invention, the polyoxypropylene (PPO) content in the poloxamer is from 30 to 90 wt%, more particularly about 70%. In one embodiment of the invention, the polyoxypropylene (PPO) molecular mass in the poloxamer is from 1000 to 5000 g/mol, more particularly about 4000. An example of a poloxamer is poloxamer 407 (Pluronic® F-127).

The compositions of the invention comprise two adhesive agents which are thermally reversible adhesive agent, i.e. agents which contribute to the adhesion of the composition and to its thermoreversibility. Thus, for the purpose of the invention "thermoreversible adhesive agent" means that the adhesive agent is capable to form a composition that is liquid at room temperature and a gel at body temperature.

In one particular embodiment, optionally in combination with one or more features of the various embodiments described above or below, one of the adhesive agents is a poloxamer and the other one is methyl cellulose.

In another particular embodiment, optionally in combination with one or more features of the various embodiments described above or below, the adhesive agents are present in an amount from 12 to 20%, more particularly from 14 to 18% by weight (wt%)

In another particular embodiment, optionally in combination with one or more features of the various embodiments described above or below, the weight ratio between the poloxamer and the other adhesive agent is from 4:1 to 25:1, more particularly from 8:1 to 12:1, more particularly from 9:1 to 11:1, even more particularly is 10:1.

The compositions of the invention comprise a cellulose ether which is methyl cellulose and a poloxamer as adhesive agents. More particularly methyl cellulose has a percentage of methoxy substitution from 25 to 33% and a weight average molecular weight from 10000 to 20000 Daltons.

In another particular embodiment, optionally in combination with one or more features of the various embodiments described above or below, the pharmaceutical or veterinary composition comprises two adhesive agents one being a poloxamer, and the other is methyl cellulose, wherein the poloxamer is present in an amount from 12 to 20%, more particularly from 14 to 18% by weight (wt%), with respect to the total weight of the composition, and the other adhesive agent is present in an amount from 0.75 to 3% by weight (wt%), more particularly from 1 to 2% by weight (wt%), with respect to the total weight of the composition.

In one particular embodiment, optionally in combination with one or more features of the various embodiments described above or below, the poloxamer is in a form other than micro and/or nanoparticles. In this embodiment, the composition of the invention is obtainable by mixing in any order a hyaluronic acid or a pharmaceutically or veterinary acceptable salt thereof, and two thermoreversible adhesive agents, being one of them a poloxamer, wherein the weight ratio between the poloxamer and the hyaluronic acid or its salt is from 60:1 to 10:1.

Thus, it also forms part of the present invention a pharmaceutical or veterinary composition suitable for the delivery of active agents comprising:

a) from 0.25 to 1.5 wt% a hyaluronic acid or a pharmaceutically or veterinary acceptable salt thereof as active agent, and
b) from 10 to 25 wt% of two thermoreversible adhesive agents, being one of the adhesive agents a poloxamer, and the other is methyl cellulose, wherein

thermoreversible means that the adhesive agent is capable to form a composition that is liquid at room temperature and a gel at body temperature,
in the absence of any further active agent,
wherein the weight ratio between the poloxamer and the hyaluronic acid or its salt is from 60:1 to 10:1;
wherein all percentages are expressed with respect to the total weight of the composition, provided that the sum of the amounts of the components is equal to or less than 100%; which is obtainable by mixing in any order a hyaluronic acid or a pharmaceutically or veterinary acceptable salt thereof, and two thermoreversible adhesive agents, being one of them a poloxamer, wherein the weight ratio between the poloxamer and the hyaluronic acid or its salt is from 60:1 to 10:1.

The above described preparation process comprising mixing the components, particularly in water or a buffer, and stirring until achieving the complete dissolution thereof is also part of the present invention.

The term "obtainable by" is used herein for defining the compositions of the invention by its preparation process and refers to the products that can be obtained through the preparation process which comprise the indicated steps as herein defined. For the purposes of the invention, the expressions "obtainable", "obtained" and similar equivalent expressions are used interchangeably and, in any case, the expression "obtainable" encompasses the expression "obtained".

In another embodiment, optionally in combination with one or more features of the various embodiments described above or below, the compositions of the invention are aqueous composition, which may be buffered. In such case when the aqueous composition contacts the target tissue or organ within the body a hydrogel is formed.

In another particular embodiment, optionally in combination with one or more features of the various embodiments described above or below, water is present in an amount from 75 to 85%, more particularly from 76 to 83%, by weight (wt%), with respect to the total weight of the composition.

The composition of the invention may also comprise further components, such as for example mineral cofactors, more particularly cofactors for Matrix metalloproteinases (MMPs). As used herein, "cofactor" refers to an agent that activates an enzyme, more particularly an endopeptidase, such as MMP.

Examples of mineral cofactors of the formulation may include zinc compounds, calcium compounds, manganese compounds, and magnesium compounds. Particularly suitable mineral cofactors are zinc cofactors such as zinc oxide, zinc gluconate, zinc amino acid chelates or mixtures thereof.

In one particular embodiment, optionally in combination with one or more features of the various embodiments described above or below, the composition comprises a mineral cofactor, more particularly a mineral cofactors for Matrix metalloproteinases (MMPs), more particularly a zinc cofactor, even more particularly zinc oxide. The cofactor may be present in the composition in an amount from 4 to 10 wt% by weight, more particularly 6 to 8% by weight, with respect to the total weight of the composition.

The compositions of the invention are biodegradable. This means that it is bioresorbed or degraded or broken down into components that are well tolerated by the body of the patient. Thus, there is no need to remove the composition of the invention once applied to the body.

In one embodiment, optionally in combination with one or more features of the various embodiments described above or below, the invention refers to topical compositions. For the purposes of the present invention, the term "topical" refers to the local administration of the composition other than systemic (i.e., parenteral and enteral) administration.

As mentioned above the base composition of the invention may also be used as a platform for the local and sustained delivery of active agents. Thus, the invention also relates to a pharmaceutical or veterinary composition comprising the base composition as defined above and a therapeutically effective amount of one or more further active agents.

The compositions of the invention do not include a topical composition comprising: from 0.6 to 1.5 wt% of a hyaluronic acid or a pharmaceutically or veterinary acceptable salt thereof, from 0.75 to 25 wt% of one or more adhesive agents, and from 1.5 to 2.5 wt% of a non-absorbable antibiotic. This specific composition is described in the PCT application PCT/EP2016/053928 filed on 25.02.2016.

In particular, the topical composition containing hyaluronic acid sodium salt (1 wt%), methylcellulose (2 wt%), Pluronic acid F127 (20 wt%), Rifaximin (2 wt%), and water (75 wt%), and the topical composition containing hyaluronic acid sodium salt (1 wt%), methylcellulose (2 wt%), Rifaximin (2 wt%), and water (95 wt%), wherein the average Molecular weight of the hyaluronic acid sodium salt is $1.5 \times 10^6 - 4 \times 10^6$ Daltons, the approximate Molecular Weight of methyl cellulose is 14000 g/mol, with methoxy substitution between 27.5-31.5% (w), and the average molecular weight of pluronic acid is 12600 Daltons, do not form part of the present invention.

Thus, in one embodiment the invention relates to a composition comprising the base composition as previously defined and a therapeutically effective amount of one or more further active agents; with the condition that the composition is other than a topical composition containing hyaluronic acid sodium salt (1 wt%), methylcellulose (2 wt%), Pluronic acid F127 (20 wt%), Rifaximin (2 wt%), and water (75 wt%), and other than a topical composition containing hyaluronic acid sodium salt (1 wt%), methylcellulose (2 wt%), Rifaximin (2 wt%), and water (95 wt%), wherein the average Molecular weight of the hyaluronic acid sodium salt is $1.5 \times 10^6 - 4 \times 10^6$ Daltons, the approximate Molecular Weight of methyl cellulose is 14000 g/mol, with methoxy substitution between 27.5-31.5% (w), and the average molecular weight of pluronic acid is 12600 Daltons.

The expression "therapeutically effective amount" as used herein, refers to the amount of the composition of the invention that, when administered, is sufficient to prevent development of, or alleviate to some extent, one or more of the symptoms of the disease which is addressed. The specific dose of the composition to obtain a therapeutic benefit may vary depending on the particular circumstances of the case.

For the purposes of the invention, each component of the composition as defined above must be pharmaceutically or veterinary acceptable in the sense of being compatible with the other ingredients of the composition. It must also be suitable for use in contact with the tissue or organ of humans and animals without excessive toxicity, irritation, allergic response, immunogenicity or other problems or complications commensurate with a reasonable benefit/risk ratio.

In one particular embodiment, optionally in combination with one or more features of the various embodiments described above or below, the composition further comprising one or more further active agents as herein defined is stable without encapsulation of the further active agent by the poloxamer. In this embodiment, the composition of the invention is obtainable by mixing in any order a hyaluronic acid or a pharmaceutically or veterinary acceptable salt thereof, two thermoreversible adhesive agents, being one of them a poloxamer, and a therapeutically effective amount of one or more further active agents, wherein the weight ratio between the poloxamer and the hyaluronic acid or its salt is from 60:1 to 10:1.

Thus, it also forms part of the present invention a pharmaceutical or veterinary composition comprising:

a) from 0.25 to 1.5 wt% a hyaluronic acid or a pharmaceutically or veterinary acceptable salt thereof as active agent,
b) from 10 to 25 wt% of two thermoreversible adhesive agents, being one of the adhesive agents a poloxamer, and the other is methyl cellulose, wherein thermoreversible means that the adhesive agent is capable to form a composition that is liquid at room temperature and a gel at body temperature, and
c) a therapeutically effective amount of one or more further active agents,

wherein the weight ratio between the poloxamer and the hyaluronic acid or its salt is from 60:1 to 10:1; wherein all percentages are expressed with respect to the total weight of the composition, provided that the sum of the amounts of the components is equal to or less than 100%; with the condition that the further active agent is other than a non-absorbable antibiotic, wherein non-absorbable means that the antibiotic is capable of providing activity only locally in the gut; wherein the composition is obtainable by mixing in any order a hyaluronic acid or a pharmaceutically or veterinary acceptable salt thereof, two thermoreversible adhesive agents, being one of them a poloxamer, and a therapeutically effective amount of one or more further active agents, wherein the weight ratio between the poloxamer and the hyaluronic acid or its salt is from 60:1 to 10:1.

The above described preparation process comprising mixing the components, particularly in water or a buffer, and stirring until achieving the complete dissolution thereof is also part of the present invention.

In one particular embodiment, optionally in combination with one or more features of the various embodiments described above or below, the active agent to be delivered by the base composition is selected from the group consisting of monoclonal antibodies (infliximab, adalimumab, vedolizumab, natalizumab, certolizumab), cytostatic drugs (irinotecan, oxaliplatin, cisplatin), antiangiogenic drugs (cetuximab, bevacizumab, axitinib, pazopanib, sutinib, vamdetanib, aflibercept), and antiinflamatory (naproxen, diclofenac, celecoxib, COX-2 inhibitors, ibuprofen, salicylates, corticosteroids, propionic acid and enolic acid derivatives drugs), antimicrobial agents, and probiotics or combinations of probiotics. Non limiting examples of probiotics that can be used include *Streptococcus, Lactobacillus, Bifidobacterium* or combinations thereof, such as for example a composition containing *Lactobacillus Reuteri* (Reuteri®, Casenbiotic®); a composition containing *Lactobacillus Acidofilus, Bifidobacterium Bifidum, Lactobacillus Bulgaricus, Streptoccocus Thermophilus* (Rotargemine®), *Lactobacillus Acidofilus,* and *Bifidobacterium Bifidum (*Casenfilus®, Infloran®), *Streptococcus thermophiles, Bifidobacterium breve, Bifidobacterium lactis, Lactobacillus acidophilus, Lactobacillus plantarum, Lactobacillus paracasei, Lactobacillus Helveticus* (VSL#3).

More particularly, the therapeutically active agent is selected from the group consisting of irinotecan or a pharmaceutically or veterinary acceptable salt thereof, bevacizumab, cetuximab, aflibercept, and infliximab.

In another particular embodiment, optionally in combination with one or more features of the various embodiments described above or below, the active agent to be delivered by the base composition is selected from the group consisting of monoclonal antibodies (infliximab, adalimumab, vedolizumab, natalizumab, certolizumab), cytostatic drugs (irinotecan, oxaliplatin, cisplatin), antiangiogenic drugs (cetuximab, bevacizumab, axitinib, pazopanib, sutinib, vamdetanib, aflibercept), antiinflamatory drugs (naproxen, diclofenac, celecoxib, COX-2 inhibitors, ibuprofen, salicylates, corticosteroids, propionic acid and enolic acid derivatives drugs), absorbable antibiotics, and probiotics or combinations of probiotics (*Streptococcus, Lactobacillus,* and *Bifidobacterium* or combinations thereof).

The term "absorbable antibiotic" is defined herein by contrast to "non-absorbable antibiotics". While absorbable antibiotics refer to compounds having antibacterial properties that show a systemic absorption, "non-absorbable antibiotics" refer to compounds having antibacterial properties which are poorly or not absorbed from the lumen, i.e., they provide activity only locally in the gut and have a negligible systemic absorption.

Non-limiting examples of absorbable antibiotics include quinolones such as norfloxacin, levofloxacin, ciprofloxacin, and the like; cephalosporins such as ceftriaxone, cefotaxime and the like; penicillins such as amoxycillin, ampicillin, and the like; and macrolides such as erythromycin, metronidazole, and the like.

In another particular embodiment, optionally in combination with one or more features of the various embodiments described above or below, the amount of active agent to be delivered by the base composition is from 0.01 to 25%, more particularly from 1 to 20%, more particularly from 1 to 15% by weight (wt%) with respect to the total weight of the composition.

A mentioned above it also forms part of the pre-loaded injection device comprising the composition as previously defined, and a kit comprising this injection device, a delivery device that is suitable to be coupled to the injection device, and instructions for use.

The injection device may be any device appropriate for administering the composition of the invention that is suitable to be coupled or connected to the delivery device. Non-limiting examples of injection devices include syringes or jet injectors.

The delivery device can be any tubular device having a lumen that is suitable to be coupled or connected to the injection device and is capable to deliver the composition of the invention to its action site. Non-limiting examples of delivery devices include catheters.

In one embodiment, optionally in combination with one or more features of the various embodiments described above or below, the delivery device has a smaller diameter than the diameter of the endoscope diameter.

For example, the compositions of the invention can be applied by using an appropriate delivery device or system such as a catheter which can be introduced via an endoscope. Thus, for this therapeutic application, the delivery device has a smaller diameter than the diameter of the endoscope.

The endoscope can be the same endoscope used to carry out the therapeutic endoscopy.

Generally, gastrointestinal endoscopes have diameter in the range of 2.8-3.4 mm and a length of 160 cm. In one particular embodiment, the delivery device has a diameter lower than 2.8 mm, more particularly, lower than 2.2 mm, and a length higher than 160 cm, more particularly higher than 200 cm. For example, the length of the delivery device may be 230 cm.

The skilled in the art will know the injection device to be chosen depending on the delivery device to be used so that the composition may be administered by using an adequate force.

For example, in one embodiment, optionally in combination with one or more features of the various embodiments described above or below, the invention relates to a delivery device, particularly a catheter, comprising the composition as previously defined, wherein the delivery device has a diameter in the range of 2.0-2.2 mm, and the injector device is a syringe. In this case, the composition may be administered by applying a force of about 2-3 atmospheres.

In another embodiment, optionally in combination with one or more features of the various embodiments described above or below, the invention relates to a delivery device, particularly a catheter, comprising the composition as previously defined, wherein the delivery device has a diameter in the range of 0.6-0.8 mm, and the injector device is a jet injector. In this case, the composition may be administered by applying a force of about more than 5 atmospheres.

As mentioned above, the base composition as defined above may be used in the treatment of mucosal lesions and/or for the prevention of complications derived from mucosal lesions.

In another embodiment, optionally in combination with one or more features of the various embodiments described above or below, the mucosal lesions are induced by thermal injury, and more particularly, thermal injury associated or caused by therapeutic endoscopy.

As used herein, thermal injury refers to an injury caused by either extreme cold or heat which alters or damages the tissue, chemical or electrical burn which alters or damages the tissue, or chemical or electrical trauma which alters or damages the tissue.

In another particular embodiment, optionally in combination with one or more features of the various embodiments described above or below, the invention relates to a composition as defined above for use in the prevention of postpolypectomy syndrome.

In another particular embodiment, optionally in combination with one or more features of the various embodiments described above or below, the invention relates to the composition as defined above for use in the treatment and/or prevention of mucosal lesions secondary to radiotherapy (actinic proctitis).

In another particular embodiment, optionally in combination with one or more features of the various embodiments described above or below, the invention relates to the composition as defined above for use in the treatment and/or prevention of mucosal lesions which are mucosal perforations, more particularly gastrointestinal perforations. More particularly, the invention relates to the composition as defined above may be used as adjuvant therapy to mechanical treatments in gastrointestinal perforations, more particularly, gastrointestinal perforations secondary to endoscopy.

Additionally, the composition of the invention is also useful as coadyuvant therapy in surgical procedures in the gastrointestinal tract, such us intestinal anastomoses, which is a surgical procedure to establish communication and restore intestinal continuity between two formerly distant portions of the intestine, after removal of a pathological condition affecting the bowel. It is also useful as sealant treatment in leaks or fistulas in the gastrointestinal tract.

As mentioned above, the compositions of the invention show a higher mucosal healing rate and a higher physiological healing, while reducing at the same time the fibrotic healing in comparison to a composition consisting only of hyaluronic acid.

As used herein, the term "physiological healing" refers to the restoration of damaged living tissue, organs and biological system to normal function. It is the process by which the cells in the body regenerate and repair to reduce the size of a damaged or necrotic area. The term "fibrotic healing" refers to the temporal and progressive deposition of fibrous tissue over the affected tissue during fibrosis. Generally, when fibrotic healing occurs, a scar is formed which may be cumbersome and vulnerable to repeated trauma.

As mentioned above, the invention also relates to a pharmaceutical or veterinary composition comprising one or more further active agents in addition to hyaluronic acid or its salt. In one aspect, the invention relates to a pharmaceutical or veterinary composition comprising one or more further active agents in addition to hyaluronic acid or its salt as defined above for use as a medicament.

In one embodiment the composition of the invention comprises an additional active agent selected from the group consisting of irinotecan or their pharmaceutically or veterinary acceptable salts, bevacizumab, infliximab, and cetuximab. In this embodiment, the composition may be used to prevent tumor recurrence in colorectal cancer. Thus, the invention relates to a pharmaceutical or veterinary composition as defined above, wherein the active agent is selected from the group consisting of irinotecan or their pharmaceutically or veterinary acceptable salts, bevacizumab, infliximab, and cetuximab, for use in the prevention of colorectal cancer recurrence. This aspect relates to the use of an active agent selected from the group consisting of irinotecan or their pharmaceutically or veterinary acceptable salts, bevacizumab, infliximab, and cetuximab, for the manufacture of a pharmaceutical or veterinary composition as defined above for the prevention of colorectal cancer recurrence.

In another embodiment the composition of the invention comprises infliximab and may be used to perform topical treatment in refractory inflammatory lesions, such as inflammatory bowel disease. Thus, the invention relates to a pharmaceutical or veterinary composition as defined above, which comprises infliximab, for use in the topical treatment in refractory inflammatory lesions, such as inflammatory bowel disease. This aspect relates to the use infliximab, for the manufacture of a pharmaceutical or veterinary composition as defined above for the topical treatment in refractory inflammatory lesions, such as inflammatory bowel disease.

The following examples and drawings are provided by way of illustration, and they are not intended to be limiting of the present invention.

**Examples**

Chemicals used:

Hyaluronic acid sodium salt (from rooster comb): Also known as: Poly(betaglucuronic acid-[1->3]-beta-N-acetylglucosamine-[1->4]); Average Molecular weight: 1.5 x 106 - 4 x 106 Daltons.

Methyl cellulose: Also known as: Methocel A®, Methylcellulose A, Methyl cellulose ether. Approximate Molecular Weight: 14000 g/mol: Cellulose, with methoxy substitution between 27.5-31.5% (w).

Pluronic® F127: Also known as: poloxamer 407, PPG-PEG-PPG; Pluronic(R)-F-68; Poly(ethylene glycol-ran-propylene glycol); Polyoxyethylene-polyoxypropylene Block Copolymer; Molecular Formula: C5H14O4; Molecular Weight: 138.16226 g/mol. Average molecular weight: 12600 Daltons.

Example 1 - Preparation of the base composition

In a 100 mL beaker, 2 g of the surfactant Pluronic F127 on 10 mL of distilled water were added and the mixture was stirred at 500 rpm until complete dissolution. Then 0.05 g of hyaluronic acid sodium salt were added and the mixture was stirred at 500 rpm for 10 minutes until complete dissolution of hyaluronic acid. Then, 0.2 g of methylcellulose was added and stirred until completely dissolved. The sample was stored in the refrigerator to remove bubbles. Thus, the following composition having a pH = 3 was obtained:

| Component | Amount | % |
|---|---|---|
| Hyaluronic acid sodium salt | 0.05 g | 0.4 |
| Methylcellulose | 0.2 g | 1.6 |
| Pluronic acid F127 | 2.0 g | 16.4 |
| Water | 10 mL | 81.6 |

Example 2 - Preparation of a composition containing infliximab

9.5 mL of the composition of example 1 were placed in a 50 mL beaker. The pH of the solution was raised from 3 to 10 by the addition of triethylamine (TEA). Finally, 0.5 mL of an infliximab stock solution having a concentration of 10 mg/mL was added. The final concentration of infliximab in the final composition was 0.5 mg/mL. Thus, the following composition was obtained:

| Component | Amount | % |
|---|---|---|
| Hyaluronic acid sodium salt | 0.048 g | 0.4 |

(continued)

| Component | Amount | % |
|---|---|---|
| Methylcellulose | 0.19 g | 1.55 |
| Pluronic acid F127 | 1.9 g | 15.7 |
| Infliximab (10 mg/mL solution) | 0.5 mL | 4.1 |
| Water | 9.5 mL | 78.25 |

Example 3 - Preparation of a composition containing irinotecan

9.35 mL of the composition of example 1 were placed in a 50 mL beaker. Then, 0.65 mL of a irinotecan stock solution having a concentration of 20 mg/mL was added. The final concentration of irinotecan in the final composition was 1.3 mg/mL. Thus, the following composition was obtained:

| Component | Amount | % |
|---|---|---|
| Hyaluronic acid sodium salt | 0.047 g | 0.39 |
| Methylcellulose | 0.19 g | 1.61 |
| Pluronic acid F127 | 1.9 g | 15.7 |
| Irinotecan (20 mg/mL solution) | 0.65 mL | 5.3 |
| Water | 9.35 mL | 77.0 |

Hydrogel characterization

Adhesion tests

A Texture Analyser TA.XT Plus was used to determine the texture properties of the hydrogels. A 40 mm (diameter) disk was compressed into the gel and redrawn. The method settings, including speed rate at 1 mm/s and distance (depth of the insertion) of 9 mm were assessed at 22 °C and 37 °C. Hydrogels of Examples 1, 2 and 3 were tested and the results shown in table 1 below were obtained:

|  | At 22°C | | At 37 °C | |
|---|---|---|---|---|
|  | Adhesion (mN/s) | SD | Adhesion (mN/s) | SD |
| Example 1 | -24.48 | 5.34 | -3992.93 | 536.21 |
| Example 2 | -44.98 | 10.69 | -2930.02 | 505.12 |
| Example 3 | -42.32 | 8.07 | -1388.14 | 233.54 |

All three examples presented a similar and very low adhesion at 22 °C. However, when the temperature was increased to 37 °C, an immediate gelification was observed. The composition comprising the drug infliximab (-2930.02 mN/s) has a value similar to the base composition (-3992.93 mN/s) adhesiveness, while the composition containing irinotecan has a lower value (-1388.14 mN/s), although still is a very high value to remain adhered to the intestinal mucosa.

Rheological assay

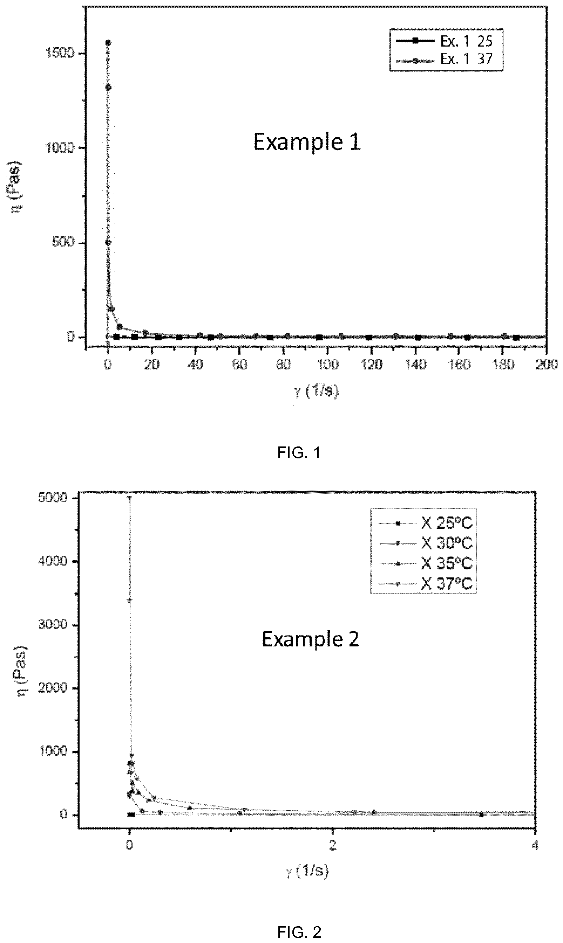

A rheological study was performed in a rheometer Haake RheoStress with a C60/1°Ti probe and a gap set of 0.053 mm. Viscosity ($\eta$) was measured as a function of shear rate ($\gamma$) of 0 to 300 s-1 at 22 °C and 37 °C. The compositions of Examples 1, 2 and 3 were tested, and the obtained results are shown in FIG. 1, 2 and 3 respectively.

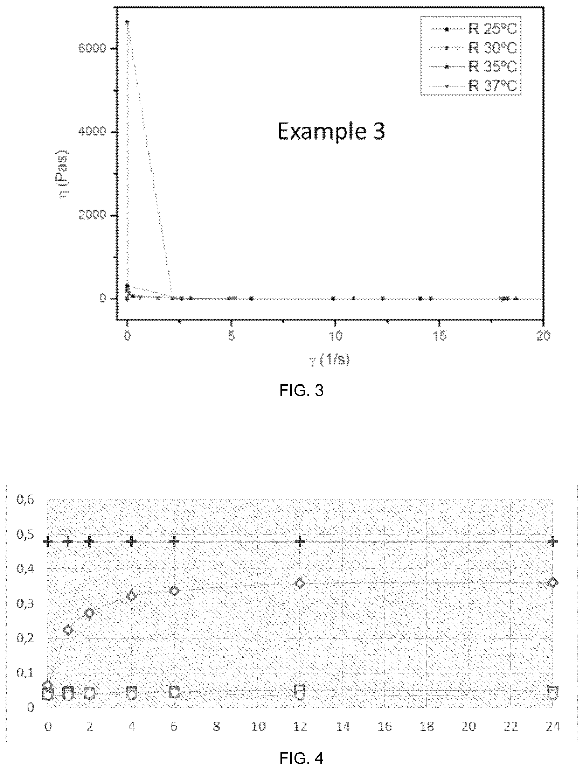

According to the results obtained, all three compositions presented a non-Newtonian fluid behaviour. A non-Newtonian fluid is a fluid whose viscosity is not defined or constant, varying with temperature and the shear stress applied to it. The hydrogel of Example 1 conformed well to the model Herschel-Bulkley rheological ($\eta = \tau0 / \gamma + K. \gamma n-1$) where $\eta$ is the apparent viscosity and $\gamma$ the shear rate. FIG. 1 shows the evolution of the represented viscosity of the composition of Example 1 according to the speed of shear. The product showed an increase in viscosity with temperature, since the initial viscosity (22 °C) increased from < 1 Pa·sec. to 1550 Pa·sec. at 37 °C. On the other hand, when the temperature of the composition containing infliximab was progressively increased, it was observed that the viscosity increased progressively < 1 Pa·sec at 25 °C to a maximum value of 5000 Pa·sec at 37 °C (FIG. 2). On the other hand, in the case of the composition containing irinotecan, the maximum viscosity (6200 Pa·sec) was observed at 30 °C. From 30 °C gel break was observed, and therefore its viscosity decreased as a result of the continuous shearing of the sample during the test and temperature ramp (FIG. 3).

Hydrogel characterization

Gelification test

The behaviour of the hydrogels of examples 2 and 3 at room temperature and at body temperature was checked. To do this, the viscosity was checked at room temperature by injecting the tested compositions through a catheter (160 cm long - 2.8 mm internal diameter). Both samples showed a fluid behaviour and suitable for injection. Then, the gelification test was performed. For this purpose, a certain volume of each of the compositions was introduced in a glass blister, then placed in an oil bath at 37 °C and stirred (200 rpm) for 10, 20 and 30 minutes. For each time, the blister was rotated 180 degrees and looked at whether the dressing had gelled or not. In both cases gelification was observed indicating that the compositions had changed their viscosity with temperature.

Stability of the composition

For the purpose of studying the stability of the composition, 0.1 mL of the base composition (example 1) were deposited in a glass preheated to 37 °C with an inclination of 60°. The distance covered by the composition before gelification was measured. Gelification was identical in fresh samples and in samples stored for 3 months, both compositions gelling instantly. Thus, the base composition of example 1 maintained its rheological properties for at least 3 months refrigerated below 8 °C.

Drug delivery

In vitro delivery drug studies, performed to analyze the kinetics of Indigo Carmine release from the example 1 composition in PBS at 37° C during 24 hours, showed the ability of the composition of the invention to release in a sustained manner substances such as indigo carmine. To do this, 50 μL of 4% Indigo carmine solution was added to 950 μL of example 1 composition, and then it was deposited in one well of a 6-well culture plate and filled with 4mL of PBS. Additionally, 50 μL of 4% indigo carmine were added to 4 mL of PBS in other well (total release control), 1 mL of example 1 composition in 4 mL of PBS in other well and 4 mL of PBS in the last well (acting, both, as negative controls). A 100 μL sample was collected of each well at times 0, 1, 2, 4, 6, 12 and 24 hours. Indigo carmine concentration in PBS medium was quantified by colorimetric analysis (FIG. 4).
During the first 6 hours, more than 70% of the drug was already delivered from the composition of example 1. In the figure, indigo carmine absorbance at 611 nm was recorded, compared to control PBS (crosses), and two negative controls: the composition of example 1 without indigo carmine (squares) and PBS without indigo carmine (circles).

Fluid dynamics assay

To assess the pressure injection of the composition (example 1), a dynamic assay was performed prefilling a catheter (150 cm long - 1.16 mm internal diameter) with 1.58 mL of the composition, attached to an infusion pump (GIP-3000 Infusion Pump) at 25 mL/min. The equation for laminar flow of the composition through a catheter is:

$$Flowrate = \frac{\pi r^4 (P - P_0)}{8 \eta l}$$

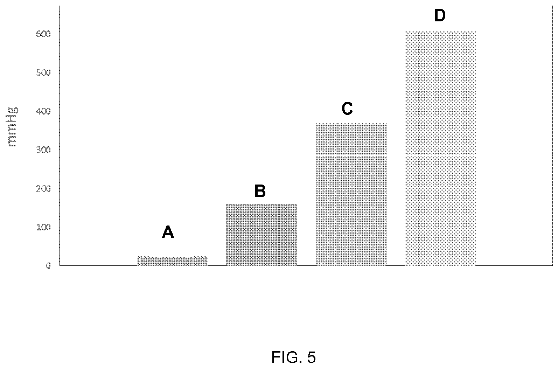

where $\pi r^4$ is the radius of the pipe; Po is the the pressure at the end of the pipe; P is the pressure needed to flush; η is the fluid's viscosity and l is the lenght of the pipe.
Pressure needed to flush the composition was recorded in mmHg. Four samples were assessed: saline (A), example 1 (C), comparative composition 1 (B), and comparative composition 2 (D). See FIG. 5.
Comparative compositions 1 and 2 correspond to the composition of example 1 with different amounts of the components. Thus, in comparative composition 1 (B) the percentage of methylcellulose and pluronic acid is 9.8% and in comparative composition 2 (D) the percentage of methylcellulose and pluronic acid is 30.4%. In both cases, the weight ratio between the pluronic acid and the hyaluronic acid sodium salt is 40:1.

The results showed that higher viscosity of the fluid needed higher pressure to flow. Pressures higher than 400 mmHg are not generally suitable to be used for fluid injections since this pressure is too high to maintain an adequate flow. On the other hand, when the comparative composition 1 (B) was placed on a plate at 37 °C it was observed that the composition did not form a gel.

Preclinical studies

Experimental model of TNBS induced Colitis

Twenty-four male Sprague-Dawley rats weighing 380-400 g (Harlan Laboratoires, Barcelona) were housed individually in polycarbonate box cages with free access to water and food (Teklad Global 2014; Harlan Laboratories Models SL, Barcelona, Spain). The protocol was approved by the Institutional Animal Care and Use Committee of Hospital Universitari Germans Trias i Pujol.

Study design:

Day -3: Colitis was induced by intrarectal administration of 30 mg TNBS (Sigma-Aldrich Corp., St. Louis, MO) in 50% ethanol.

Day 0: Animals were randomized into three treatment groups as follows:

- Group 1: 8 rats with TNBS-induced colitis treated with the composition of example 2 (infliximab 0.5 mg/mL).
- Group 2: 8 rats with TNBS-induced colitis treated with the composition base of example 1.
- Group 3: 8 rats with TNBS-induced colitis with no treatment (control).

Day 3: macroscopic follow-up (colonoscopy).
Day 8: macroscopic follow-up and sacrifice
Ponderal evolution and appearance of stool were recorded during the study.

Procedures:

TNBS was rectally instilled via a female urinary catheter (DCT Ch 10, Servoprax GmbH, Wesel, Germany). After removal of residual rectal fecal pellets, the catheter was advanced approximately to the splenic flexure. After instillation, the rats were held with the head down for one minute to prevent TNBS from leaking out. The colitis was evaluated with full colonoscopy performed using an endoscope Olympus 260 Lucera -HDTV / NBI / AFI with an outer diameter of 4.9 mm and a working channel of 2 mm, where the presence of ulcers (size and position) was recorded and proceed to the administration of the tested compositions through the working channel of the endoscope on the lesions.

Macroscopic colitis severity was assessed using video endoscopy, a method that provides a robust clinical readout of disease severity. Images were scored by a pathologist without any information regarding the group: 0 = normal, 1 = loss of vascularity, 2 = loss of vascularity and friability, 3 = friability and mucosal erosions, and 4 = ulcerations and bleeding. After sacrifice, the colon was collected and rinsed with ice-cold Krebs solution. The colon was opened longitudinally and pinned out on a Petri dish to examine colonic mucosa. The mucosal surface of the distal colon was inspected with a binocular microscope (Harvard Apparatus; Panlab, Barcelona, Spain). Full-thickness samples of 4 cm were taken from ulcerated and healthy areas. Segments were fixed in 4% formaldehyde for 24 h, embedded in paraffin, and cross sections of 5 mm were stained with hematoxylin and eosin. Histologic sections were examined using a conventional microscope (Olympus). Histological study of the specimens was assessed according to damage score.

The following results were obtained:
The treatment with the composition of example 2 significantly improved the clinical condition of the animals (weight evolution, and stool appearance) when compared to the control group. The use of the composition of example 1 also improved, but not significantly compared to controls. Similarly, the weight of the colon, was also lower in animals treated with Tri-Bio + IFX when compared to controls.

Histologic score also showed that treatment significantly reduced the ulcer and the presence of necrosis and fibrosis. Clinical evolution of the animals showed that ponderal restoration was significantly better with example 2 (IFX delivery platform). In this sense, stool appearance and colon weight were normalized with example 2. Mucosal healing, that confirms clinical efficacy, demonstrates a protective effect of the platform alone, but even a better outcome when it is used as a drug delivery system. These results show than bioadhesive platform induce mucosal restoration with clinical improvements. These results confirm than bioadhesive platform induce mucosal restoration with clinical improvements.

|  | Example 2 | Example 1 | Control |
|---|---|---|---|
| Ponderal evolution (% Variation) | | | |
| Day 0 | -7.9±2.1 | -6.4±1.8 | -9.4±1.8 |
| Day 3 | -5.2±2.7* | -11.4±5.2 | -15.7±3.6 |
| Day 8 (sacrifice) | -2.2±2.3* | -8.9±4.1 | -17.1±7.5 |
| Macroscopic features | | | |

|  | Example 2 | Example 1 | Control |
|---|---|---|---|
| Ponderal evolution (% Variation) | | | |
| Stool appearance | Normal | liquid | Presence of blood |
| Colon weight (g/cm) | 0.24±0.79* | 0.48±0.03 | 0.65±0.41 |
| Histologic score (descriptive) | | | |
| Ulceration (area) | 2.1±2.0* | 9.8±4.6 | 14.2±11.1 |
| Necrosis | 0.6±0.8* | 1.8±0.4 | 2.0±0.0 |
| Fibrosis | 0.5±0.8* | 1.4±0.5 | 1.3±0.6 |
| *p<0.05 vs. control | | | |

Degradation test

MATERIALS

• 1 Sprague-Dawley male rat.
• 3 blood Agar plates (Columbia agar + 5% sheep blood, Biomerieux, France). This culture media is highly nutritious and therefore adapted to the culture of most bacterial species, regardless of their metabolism.
• The following compositions were tested:
A: Composition of the invention of Example 1 having the following composition

| Component | Amount (% w/w) |
|---|---|
| Hyaluronic acid sodium salt | 0.4 |
| Methylcellulose | 1.6 |
| Pluronic acid F127 | 16.4 |
| Water | 81.6 |
| Total | 100 |

• B: Comparative composition 3 (without Pluronic F127)

| Component | Amount (% w/w) |
|---|---|
| Hyaluronic acid sodium salt | 0.4 |
| Methylcellulose | 3.2 |
| Water | 95 |
| Total | 100 |

• C: Comparative composition 4 (without Methylcellulose)

| Component | Amount (% w/w) |
|---|---|
| Hyaluronic acid sodium salt | 0.4 |
| Pluronic acid F127 | 16.4 |
| Water | 83.2 |
| Total | 100 |

• NaCl 0.9% (w/v) in water (saline)
• Indigo carmine solution (4%)

METHODS

Rat was anesthetized by isoflurane inhalation (1.5% with 98% $O_2$) and placed in "Trendelenburg" position. Twenty mL of saline were intracolonically instilled trough a catheter, and 2 mL were recovered in order to obtain colonic bacterial flora (colonic lavage). 100 $\mu$L of colonic lavage was plated on plates 1, 2 and 3 and cultured at 37 °C for 24h. At this time all plate surfaces were full of bacterial colonies and ready to be used. 50 $\mu$L of indigo carmine solution was added to 950 $\mu$L of each composition (A, B and C). 1 mL of the composition of example 1 (A) was added to plate number 1. 1 mL of the comparative composition 2 (without pluronic F127) (B) was added to plate number 2. 1 mL of the comparative composition 3 (without methylcellulose) (C) was added to plate number 3. The three plates were incubated at 37°C for 72 hours. They were photographed at t = 0, t= 6 h, t = 24h, t = 36h and t = 48h.

RESULTS:

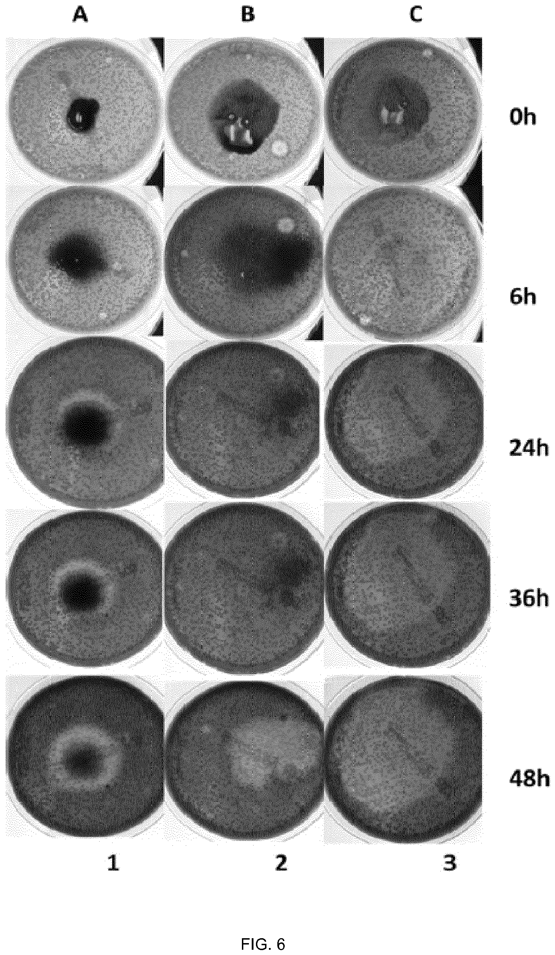

The results are shown in FIG. 6.

- Plate 1: The composition of example 1 (A) deposited on the plate seeded with colonic lavage maintained, with very low degradation, its integrity for at least 48h. The red to yellow color change was due to the acidification of the agar medium caused by the fermentation of the bacteria that are degrading the agar.

- Plate 2: The comparative composition 3 (without Pluronic F127) (B) deposited on the plate seeded with colonic lavage, showed a degradation of its integrity faster than the example 1 showing a high degradation at 24h and a total degradation at 48h. Also, the acidification of agar by bacterial fermentation could be observed at 24 hours and 72 hours.

- Plate 3: The comparative composition 4 (without methylcellulose) (C) deposited on the plate seeded with colonic lavage, showed the fastest degradation of its integrity, being almost complete in only 6 hours.

CONCLUSIONS:

According to the obtained results, the simultaneous presence of the two adhesive agents (Pluronic F127 and methylcellulose) is capable to lengthening its half-life, as compared to comparative examples 3 and 4, without pluronic or methylcellulose, respectively, allowing it to be adhered to the targeted site for a longer period for the local delivery of active agents to the gastrointestinal tract.

**Citation List**

CA2703807
US20060280797

**Claims**

1. A base pharmaceutical or veterinary composition suitable for the delivery of active agents comprising:

a) from 0.25 to 1.5 wt% a hyaluronic acid or a pharmaceutically or veterinary acceptable salt thereof as active agent, and

b) from 10 to 25 wt% of two thermoreversible adhesive agents, wherein one of the adhesive agents is a poloxamer and the other is methyl cellulose,

wherein thermoreversible means that the adhesive agent is capable to form a composition that is liquid at room temperature and a gel at body temperature,

in the absence of any further active agent,

wherein the weight ratio between the poloxamer and the hyaluronic acid or its salt is from 60:1 to 10:1; and

wherein all percentages are expressed with respect to the total weight of the composition, provided that the sum of the amounts of the components is equal to or less than 100%.

2. The composition according to claim 1, wherein the weight ratio between the poloxamer and the hyaluronic acid or its salt is from 60:1 to 20:1.

3. The composition according to any of the claims 1-2, wherein the hyaluronic acid or a pharmaceutically or veterinary acceptable salt is hyaluronic acid sodium salt.

4. The composition according to any of the claims 1-3, wherein the hyaluronic acid or its pharmaceutically or veterinary acceptable salt is present in an amount from 0.3 to 0.8 wt% with respect to the total weight of the composition.

5. The composition according to any of the claims 1-4, wherein the weight ratio between the poloxamer and methyl cellulose is from 4:1 to 25:1, more particularly from 8:1 to 12:1.

6. The composition according to any of the claims 1-5, wherein methyl cellulose is present in an amount from 0.75 to 3.0 wt% and the poloxamer is present in an amount from 12 to 20 wt% with respect to the total weight of the composition,

7. The composition according to any of the claims 1-6, which is an aqueous composition.

8. A pharmaceutical or veterinary composition suitable for the delivery of active agents comprising:

a) from 0.25 to 1.5 wt% a hyaluronic acid or a pharmaceutically or veterinary acceptable salt thereof as active agent,

b) from 10 to 25 wt% of two thermoreversible adhesive agents, wherein one of the adhesive agents is a poloxamer and the other is methyl cellulose, wherein thermoreversible means that the adhesive agent is capable to form a composition that is liquid at room temperature and a gel at body temperature,

c) a therapeutically effective amount of one or more further active agents;

wherein the weight ratio between the poloxamer and the hyaluronic acid or its salt is from 60:1 to 10:1;

wherein all percentages are expressed with respect to the total weight of the composition, provided that the sum of the amounts of the components is equal to or less than 100%;

with the condition that the further active agent is other than a non-absorbable antibiotic, wherein non-absorbable means that the antibiotic is capable of providing activity only locally in the gut.

9. An injection device comprising the composition as defined in any of the claims 1-8.

10. A kit comprising the injection device as defined in claim 9, and a delivery device suitable to be coupled to the injection device.

11. A pharmaceutical or veterinary composition as defined in any of the claims 1-7, for use in the topical treatment of mucosal lesions and/or for the prevention of complications derived from mucosal lesions.

12. A pharmaceutical or veterinary composition as defined in claim 8, wherein the further active agent is selected from the group consisting of irinotecan and their pharmaceutically or veterinary acceptable salts, bevacizumab, cetuximab, aflibercept, and infliximab, for use in the prevention of colorectal cancer recurrence.

**Patentansprüche**

1. Eine pharmazeutische oder tiermedizinische Basenzusammensetzung, die zur Abgabe von Wirkstoffen geeignet ist, umfassend:

   a) von 0,25 bis 1,5 Gew.-% einer Hyaluronsäure oder eines pharmazeutisch oder tiermedizinisch akzeptablen Salzes davon als Wirkstoff, und
   b) von 10 bis 25 Gew.-% von zwei thermoreversiblen Klebemitteln, wobei eines der Klebemittel ein Poloxamer ist und das andere Methylcellulose ist,
   wobei thermoreversibel bedeutet, dass das Klebemittel in der Lage ist, eine bei Raumtemperatur flüssige Zusammensetzung und ein Gel bei Körpertemperatur zu bilden,
   in Abwesenheit eines weiteren Wirkstoffs,
   wobei das Gewichtsverhältnis zwischen dem Poloxamer und der Hyaluronsäure oder ihrem Salz von 60:1 bis 10:1 beträgt; und
   wobei alle Prozentsätze in Bezug auf das Gesamtgewicht der Zusammensetzung ausgedrückt sind, unter der Voraussetzung, dass die Summe der Mengen der Komponenten gleich oder kleiner als 100 % ist.

2. Die Zusammensetzung nach Anspruch 1, wobei das Gewichtsverhältnis zwischen dem Poloxamer und der Hyaluronsäure oder ihrem Salz von 60:1 bis 20:1 beträgt.

3. Die Zusammensetzung nach einem der Ansprüche 1 bis 2, wobei die Hyaluronsäure oder ein pharmazeutisch oder tiermedizinisch akzeptables Salz ein Natriumsalz von Hyaluronsäure ist.

4. Die Zusammensetzung nach einem der Ansprüche 1 bis 3, wobei die Hyaluronsäure oder ihr pharmazeutisch oder tiermedizinisch akzeptables Salz in einer Menge von 0,3 bis 0,8 Gew.-% bezogen auf das Gesamtgewicht der Zusammensetzung vorliegt.

5. Die Zusammensetzung nach einem der Ansprüche 1 bis 4, wobei das Gewichtsverhältnis zwischen dem Poloxamer und der Methylcellulose von 4:1 bis 25:1, insbesondere von 8:1 bis 12:1 beträgt.

6. Die Zusammensetzung nach einem der Ansprüche 1 bis 5, wobei die Methylcellulose in einer Menge von 0,75 bis 3,0 Gew.-% vorliegt und das Poloxamer in einer Menge von 12 bis 20 Gew.-%, bezogen auf das Gesamtgewicht der Zusammensetzung, vorliegt.

7. Die Zusammensetzung nach einem der Ansprüche 1 bis 6, welche eine wässrige Zusammensetzung ist.

8. Eine pharmazeutische oder tiermedizinische Zusammensetzung, die zur Abgabe von Wirkstoffen geeignet ist, umfassend:

   a) von 0,25 bis 1,5 Gew.-% einer Hyaluronsäure oder eines pharmazeutisch oder tiermedizinisch akzeptablen Salzes davon als Wirkstoff,
   b) von 10 bis 25 Gew.-% von zwei thermoreversiblen Klebemitteln, wobei eines der Klebemittel ein Polaxamer**NOTA** ist und das andere Methylcellulose ist, wobei thermoreversibel bedeutet, dass das Klebemittel in der Lage ist, eine bei Raumtemperatur flüssige Zusammensetzung und ein Gel bei Körpertemperatur zu bilden,
   c) eine therapeutisch wirksame Menge von einem oder mehreren weiteren Wirkstoffen;
   wobei alle Prozentsätze in Bezug auf das Gesamtgewicht der Zusammensetzung ausgedrückt sind, unter der Voraussetzung, dass die Summe der Mengen der Komponenten gleich oder kleiner als 100 % ist;
   mit der Bedingung, dass der weitere Wirkstoff ein anderer als ein nicht resorbierbares Antibiotikum ist, wobei nicht resorbierbar bedeutet, dass das Antibiotikum in der Lage ist, Aktivität nur lokal im Darm bereitzustellen.

9. Eine Injektionsvorrichtung umfassend die Zusammensetzung wie in einem der Ansprüche 1 bis 8 definiert.

10. Ein Kit umfassend die Injektionsvorrichtung wie in Anspruch 9 definiert und eine Abgabevorrichtung, die geeignet ist, an die Injektionsvorrichtung gekoppelt zu werden.

11. Eine pharmazeutische oder tiermedizinische Zusammensetzung wie in einem der Ansprüche 1 bis 7 definiert, zur Verwendung bei der topischen Behandlung von Schleimhautläsionen und/oder zur Prävention von Komplikationen, die von Schleimhautläsionen abgeleitet sind.

**12.** Eine pharmazeutische oder tiermedizinische Zusammensetzung wie in Anspruch 8 definiert, wobei der weitere Wirkstoff ausgewählt ist aus der Gruppe bestehend aus Irinotecan und ihren pharmazeutisch oder tiermedizinisch akzeptablen Salzen, Bevacizumab, Cetuximab, Aflibercept und Infliximab, zur Verwendung bei der Prävention des Wiederauftretens von kolorektalem Krebs.

**Revendications**

**1.** Une composition pharmaceutique ou vétérinaire de base appropriée pour l'administration d'agents actifs comprenant :

a) de 0,25 à 1,5 % en poids d'un acide hyaluronique ou d'un sel pharmaceutiquement ou vétérinaire acceptable de celui-ci en tant qu'agent actif, et
b) de 10 à 25 % en poids de deux agents adhésifs thermoréversibles, dans laquelle l'un des agents adhésifs est un poloxamère et l'autre étant la méthylcellulose,
dans laquelle thermoréversible signifie que l'agent adhésif est capable de former une composition qui est liquide à température ambiante et un gel à la température du corps,
en l'absence de tout autre agent actif,
dans laquelle le rapport de poids entre le poloxamère et l'acide hyaluronique ou son sel est de 60:1 à 10:1 ; et
dans laquelle tous les pourcentages sont exprimés par rapport au poids total de la composition, à condition que la somme des quantités des composants soit égale ou inférieure à 100 %.

**2.** La composition selon la revendication 1, dans laquelle le rapport en poids entre le poloxamère et l'acide hyaluronique ou son sel est de 60:1 à 20:1.

**3.** La composition selon l'une quelconque des revendications 1 à 2, dans laquelle l'acide hyaluronique ou un sel pharmaceutiquement ou vétérinaire acceptable est un sel de sodium d'acide hyaluronique.

**4.** La composition selon l'une quelconque des revendications 1 à 3, dans laquelle l'acide hyaluronique ou son sel pharmaceutiquement ou vétérinaire acceptable est présent en une quantité de 0,3 à 0,8 % en poids par rapport au poids total de la composition.

**5.** La composition selon l'une quelconque des revendications 1 à 4, dans laquelle le rapport en poids entre le poloxamère et la méthylcellulose est de 4:1 à 25:1, plus particulièrement de 8:1 à 12:1.

**6.** La composition selon l'une quelconque des revendications 1 à 5, dans laquelle la méthylcellulose est présente en une quantité de 0,75 à 3,0 % en poids et le poloxamère est présent en une quantité de 12 à 20 % en poids par rapport au poids total de la composition.

**7.** La composition selon l'une quelconque des revendications 1 à 6, qui est une composition aqueuse.

**8.** Une composition pharmaceutique ou vétérinaire appropriée pour l'administration d'agents actifs comprenant :

a) de 0,25 à 1,5 % en poids d'un acide hyaluronique ou d'un sel pharmaceutiquement ou vétérinaire acceptable de celui-ci en tant qu'agent actif,
b) de 10 à 25 % en poids de deux agents adhésifs thermoréversibles, dans laquelle l'un des agents adhésifs est un poloxamère et l'autre étant la méthylcellulose, dans laquelle thermoréversible signifie que l'agent adhésif est capable de former une composition qui est liquide à la température ambiante et un gel à la température du corps,
c) une quantité thérapeutiquement efficace d'un ou plusieurs autres agents actifs ; dans laquelle tous les pourcentages sont exprimés par rapport au poids total de la composition, à condition que la somme des quantités des composants soit égale ou inférieure à 100 % ;
avec la condition que l'autre agent actif soit autre qu'un antibiotique non absorbable, dans laquelle non absorbable signifie que l'antibiotique est capable de fournir de l'activité uniquement localement dans l'intestin.

**9.** Un dispositif d'injection comprenant la composition telle que définie dans l'une quelconque des revendications 1 à 8.

**10.** Un kit comprenant le dispositif d'injection tel que défini dans la revendication 9, et un dispositif d'administration

approprié pour être couplé au dispositif d'injection.

11. Une composition pharmaceutique ou vétérinaire telle que définie dans l'une quelconque des revendications 1 à 7, pour l'utilisation dans le traitement topique de lésions des muqueuses et/ou pour la prévention de complications dérivées de lésions des muqueuses.

12. Une composition pharmaceutique ou vétérinaire telle que définie dans la revendication 8, dans laquelle l'autre agent actif est choisi dans le groupe constitué par l'irinotécan et ses sels pharmaceutiquement ou vétérinaires acceptables, le bévacizumab, le cétuximab, l'aflibercept et l'infliximab, pour son utilisation dans la prévention de la récurrence du cancer colorectal.

FIG. 1

FIG. 2

FIG. 3

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 16382365
- CA 2703807
- US 20060280797 A
- EP 2016053928 W

**Non-patent literature cited in the description**

- **WANG et al.** *Carbohydrate Polymers,* 2015, vol. 137, 9-18